# EUROPEAN PATENT SPECIFICATION

(11) **EP 0 746 625 B1**
(45) Date of publication and mention of the grant of the patent: **31.03.2004**
(21) Application number: 94901245.4
(22) Date of filing: 03.11.1993
(51) Int. Cl.: C12N 15/86, C12N 15/87, C12N 15/62, C12N 5/10, C12N 7/01, C07K 14/15, C07K 14/47, A61K 48/00

(54) **TARGETABLE VECTOR PARTICLES**
ERZIELBARE VEKTORENPARTIKEL
PARTICULES VECTORIELLES POUVANT ETRE CIBLEES

(30) Priority: 09.11.1992 US 973307
(43) Date of publication of application: 11.12.1996
(73) Proprietor: THE UNITED STATES GOVERNMENT as represented by THE SECRETARY OF THE DEPARTMENT OF HEALTH AND HUMAN SERVICES, Bethesda, MD 20878 (US)
(72) Inventor: ANDERSON, W., French, San Marino, CA 91108 (US); BALTRUCKI, Leon F., W. Hartford, CT 06107-1103 (US); MASON, James, M., Laurel, MD 20707 (US)
(74) Representative: Grubb, Philip William
(86) International application number: PCT/US1993/010522
(87) International publication number: WO 1994/011524

(56) References cited:
- WO-A-90/12087
- WO-A-91/02805
- WO-A-92/05266
- WO-A-92/06180
- WO-A-92/14829
- WO-A-93/00103
- WO-A-93/05147
- WO-A-93/14188
- WO-A-93/20221
- WO-A-94/05780
- WO-A-94/06920
- BIOCHEM. AND BIOPHYS. RES. COMMUNICATIONS, vol. 178, no. 2, 1991, ACADEMIC PRESS, NEW YORK, US, pages 507-513, XP002025478 B.K. RAY AND A. RAY: "Molecular cloning and nucleotide sequence of complementary DNA encoding rabbit alpha1-acid glycoprotein"
- JOURNAL OF VIROLOGY, Volume 61, No. 5, issued May 1987, M.A. BENDER et al., "Evidence that the Packaging Signal of Moloney Murine Leukemia Virus Extends into the gag Region", pages 1639-1646.
- BIOTECHNIQUES, Volume 7, No. 9, issued 1989, A.D. MILLER et al., "Improved Retroviral Vectors for Gene Transfer and Expression", pages 980-990.

## Description

This invention relates to "targetable" vector particles. More particularly, this invention relates to vector particles which include a receptor binding region that binds to a receptor of a target cell of a human or non-human animal.

Vector particles are useful agents for introducing gene(s) or DNA (RNA) into a cell, such as a eukaryotic cell. The gene(s) is controlled by an appropriate promoter. Examples of vectors which may be employed to generate vector particles include prokaryotic vectors, such as bacterial vectors; eukaryotic vectors, including fungal vectors such as yeast vectors; and viral vectors such as DNA virus vectors, RNA virus vectors, and retroviral vectors. Retroviral vectors which have been employed for generating vector particles for introducing genes or DNA (RNA) into a cell include Moloney Murine Leukemia Virus, spleen necrosis virus, and vectors derived from retroviruses such as Rous Sarcoma Virus and Harvey Sarcoma Virus. The term "introducing" as used herein encompasses a variety of methods of transferring genes or DNA (RNA) into a cell, such methods including transformation, transduction, transfection, and infection.

Vector particles have been used for introducing DNA (RNA) into cells for gene therapy purposes. In general, such a procedure involves obtaining cells from a patient and using a vector particle to introduce desired DNA (RNA) into the cells and then providing the patient with the engineered cells for a therapeutic purpose. It would be desirable to provide alternative procedures for gene therapy. Such an alternative procedure would involve genetically engineering cells in vivo. In such a procedure, a vector particle which includes the desired DNA (RNA) would be administered directly to the target cells of a patient in vivo.

It is therefore an object of the present invention to provide gene therapy by introduction of a vector particle, such as, for example, a retroviral vector particle, directly into a desired target cell of a patient.

In accordance with an aspect of the present invention, there is provided a retroviral vector particle which includes a receptor binding region or ligand that binds to a receptor of a target cell. The receptor of the target cell is a receptor other than the amphotropic cell receptor.

Retroviruses have an envelope protein which contains a receptor binding region. Applicants have found that retroviruses can be made "targetable" to a specific type of cell if the receptor binding region of the retrovirus, which may be amphotropic, ecotropic, or xenotropic, among other types, is modified such that the receptor binding region of the envelope protein includes a receptor binding region which binds to a receptor of a target cell. For example, at least a portion of the receptor binding region and a portion or all of the hinge region of the envelope protein of the retrovirus is deleted and replaced with a receptor binding region or a ligand which binds to a receptor of a target cell. Thus. there is provided a retroviral vector wherein at least a portion of the DNA (RNA) which encodes the receptor binding region and a portion or all of the hinge region of the envelope protein of the retrovirus has been deleted and replaced with DNA (RNA) encoding a receptor binding region or a ligand which binds to a receptor of a target cell.

In one embodiment, the retrovirus is a murine leukemia virus.

The envelope of murine leukemia viruses includes a protein known as gp70. Such viruses can be made "targetable" to a specific type of cell if a portion of the gp70 protein is deleted and replaced with a receptor binding region or a ligand which binds to a receptor of a target cell. Thus, in a preferred embodiment, there is provided a retroviral vector wherein a portion, but not all, of the DNA (RNA) encoding gp70 protein has been deleted and replaced with DNA (RNA) encoding a receptor binding region or a ligand which binds to a receptor of a target cell.

In general, gp70 protein includes the following regions: (i) the secretory signal or "leader" sequence; (ii) the receptor binding domain; (iii) the hinge region; and (iv) the body portion. Preferably, at least a portion of the DNA (RNA) encoding the receptor binding domain of gp70 protein and a portion or all of the DNA (RNA) encoding the hinge region of gp70 protein is deleted and replaced with DNA (RNA) encoding a receptor binding region or a ligand which binds to a receptor of a target cell. In another embodiment, DNA (RNA) encoding the entire receptor binding domain of gp70 protein, plus all or a portion of the DNA (RNA) encoding the hinge region of gp70 protein is deleted and replaced with DNA (RNA) encoding a receptor binding region or a ligand of a target cell.

The gp70 protein may be derived from an ecotropic murine leukemia virus, a xenotropic murine leukemia virus, or an amphotropic murine leukemia virus. Ecotropic gp70 (or eco gp70) (SEQ ID NO:1) is a protein having 469 amino acids, and is encoded by (SEQ ID:2). Amino acid residues 1-33 constitute the leader sequence; amino acid residues 34-263 constitute the receptor binding domain; amino acid residues 264-312 constitute the hinge region; and amino acid residues 313-469 constitute the body portion. Preferably, DNA (RNA) encoding at least a portion of the receptor binding region and a portion or all of the hinge region is removed and replaced with DNA (RNA) encoding a receptor binding region or a ligand which binds to a receptor of a target cell. More preferably, DNA (RNA) encoding some or all of amino acid residues 34 to 312 (i.e., the receptor binding domain and the hinge region) is removed and replaced with DNA (RNA) encoding a receptor binding region or a ligand which binds to a receptor of a target cell..

Xenotropic gp70 (or xeno gp70) (SEQ ID NO:3) has 443 amino acid residues and is encoded by (SEQ ID NO:4). Amino acid residues 1-30 constitute the leader sequence; amino acid residues 31-232 constitute the receptor binding domain; amino acid residues 233-286 constitute the hinge region; and amino acid residues 287-443 constitute the body portion. Preferably, DNA (RNA) encoding at least a portion of the receptor binding region and a portion or all of the hinge region is removed and replaced with DNA (RNA) encoding a receptor binding region or a ligand which binds to a receptor of a target cell. More preferably, DNA (RNA) encoding some or all of amino acid residues 31 to 286 is removed and replaced with DNA (RNA) encoding a receptor binding region or a ligand which binds to a receptor of a target cell.

Target cells to which the retroviral vector particle may bind include, but are not limited to, liver cells, T-cells, lymphocytes, endothelial cells, T4 helper cells, and macrophages. In one embodiment, the retroviral vector particle binds to a liver cell, and in particular to hepatocytes. To enable such binding, the retroviral vector particle contains a chimeric protein encoded by DNA (RNA) in which at least a portion of the DNA (RNA) encoding the receptor binding domain of gp70 protein is removed and is replaced with DNA (RNA) which encodes a protein which binds to an asialoglycoprotein receptor (or ASG-R) of hepatocytes.

Proteins which bind to the asialoglycoprotein receptor of liver cells include, but are not limited to, asialoglycoproteins such as. for example, alpha-1-acid glycoprotein (AGP), also known as orosomucoid, and asialofetuin. AGP is a natural high-affinity ligand for ASG-R. The asialoglycoprotein receptor, or ASG-R, is expressed only by hepatocytes. The receptor is present at about 3x10⁵ copies per cell, and such receptors have a high affinity for asialoglycoproteins such as AGP. Thus, the engineering of retroviral vector particles to contain asialoglycoprotein in place of the natural receptor binding domain of gp70 generates retroviral vector particles which bind to the asialoglycoprotein receptor of hepatocytes, which provides for an efficient means of transferring genes of interest to liver cells.

Cell lines which generate retroviral vector particles that are capable of targeting the hepatocyte's asialoglycoprotein receptor without the removal of the particle's terminal sialic acid groups by neuraminidase treatment, can be developed by selection with the cytotoxic lectin, wheat germ agglutinin (WGA). Cell lines which express the retroviral proteins gag and pol become retroviral vector packaging cell lines after they are transfected with the plasmids encoding chimeric envelope genes. These cell lines express the corresponding chimeric qp 70 glycoproteins. Upon exposure to successively higher concentrations of WGA, the outgrowth of cells which synthesize glycoproteins that lack terminal sialic acid groups, is favored. (Stanley, et al., Somatic Cell Genetics, Vol. 3, pgs. 391-405 (1977)). This selection permits the isolation of cells which synthesize oligosaccharides terminating in galactosyl sugar groups. Such cells will allow the construction of packaging cell lines that are capable of generating retroviral vector particles which target the asialoglycoprotein receptor. It is also possible to select subpopulations of packaging cells which have other distinct glycotypes, such cells yielding viral vectors that potentially are capable of targeting cells other than hepatocytes. Macrophages, for example, express unique, high-mannose receptors. The PHA-resistant subpopulation will have N-linked oligosaccharides which terminate in high-mannose groups (Stanley, et al., In Vitro, Vol. 12, pgs. 208-215 (1976)). Therefore, such a cell population will be capable of producing viral vector particles capable of targeting macrophoges via this receptor. Cells with mutant glycotypes which synthesize other novel oligosaccharides after selection with other cytotoxic lectins may also prove to be useful in targeting vector particles to other cell types such as lymphocytes or endothelial cells.

In another embodiment, the receptor binding region is a receptor binding region of a human virus. In one embodiment, the receptor binding region of a human virus is a hepatitis B virus surface protein binding region, and the target cell is a liver cell.

In another embodiment, the receptor binding region of a human virus is the gp46 protein of HTLV-I virus, and the target cell is a T-cell.

In yet another embodiment, the receptor binding region of a human virus is the HIV gp120 CD4 binding region, and the target cell is a T4 helper cell.

In one embodiment, the retroviral vector may be of the LN series of vectors, as described in Bender, et al., J. Virol., Vol. 61, pgs. 1639-1649 (1987), and Miller, et al., Biotechniques, Vol. 7, pgs. 98-990 (1989).

In another embodiment, the retroviral vector includes a multiple restriction enzyme site, or multiple cloning site. The multiple cloning site includes at least four cloning, or restriction enzyme sites, wherein at least two of the sites have an average frequency of appearance in eukaryotic genes of less than once in 10,000 base pairs; i.e., the restriction product has an average size of at least 10,000 base pairs.

In general, such restriction sites, also sometimes hereinafter referred to as "rare" sites, which have an average frequency of appearance in eukaryotic genes of less than once in 10,000 base pairs, contain a CG doublet within their recognition sequence, such doublet appearing particularly infrequently in the mammalian genome. Another measure of rarity or scarcity of a restriction enzyme site in mammals is its representation in mammalian viruses, such as SV40. In general, an enzyme whose recognition sequence is absent in SV40 may be a candidate for being a "rare" mammalian cutter.

Examples of restriction enzyme sites having an average frequency of appearance in eukaryotic genes of less than once in 10,000 base pairs include, but are not limited to the NotI, SnaBI, SalI, XhoI, ClaI, SacI, EagI, and SmaI sites. Preferred cloning sites are selected from the group consisting of NotI, SnaBI, SalI, and XhoI.

Preferably, the multiple cloning site has a length no greater than about 70 base pairs, and preferably no greater than about 60 base pairs. In general, the multiple restriction enzyme site, or multiple cloning site is located between the 5' LTR and 3' LTR of the retroviral vector. The 5' end of the multiple cloning site is no greater than about 895 base pairs from the 3' end of the 5' LTR, preferably at least about 375 base pairs from the 3' end of the 5' LTR. The 3' end of the multiple cloning site is no greater than about 40 base pairs from the 5' end of the 3' LTR, and preferably at least 11 base pairs from the 5' end of the 3' LTR.

Such vectors may be engineered from existing retroviral vectors through genetic engineering techniques known in the art such that the retroviral vector includes at least four cloning sites wherein at least two of the cloning sites are selected from the group consisting of the NotI, SnaBI, SalI, and XhoI cloning sites. In a preferred embodiment, the retroviral vector includes each of the NotI, SnaBI, SalI, and XhoI cloning sites.

Such a retroviral vector may serve as part of a cloning system for the transfer of genes to such retroviral vector. Thus, there may be provided a cloning system for the manipulation of genes in a retroviral vector which includes a retroviral vector including a multiple cloning site of the type hereinabove described, and a shuttle cloning vector which includes at least two cloning sites which are compatible with at least two cloning sites selected from the group consisting of NotI, SnaBI, SalI, and XhoI located on the retroviral vector. The shuttle cloning vector also includes at least one desired gene which is capable of being transferred from said shuttle cloning vector to said retroviral vector.

The shuttle cloning vector may be constructed from a basic "backbone" vector or fragment to which are ligated one or more linkers which include cloning or restriction enzyme recognition sites. Included in the cloning sites are the compatible, or complementary cloning sites hereinabove described. Genes and/or promoters having ends corresponding to the restriction sites of the shuttle vector may be ligated into the shuttle vector through techniques known in the art.

The shuttle cloning vector may be employed to amplify DNA sequences in prokaryotic systems. The shuttle cloning vector may be prepared from plasmids generally used in prokaryotic systems and in particular in bacteria. Thus, for example, the shuttle cloning vector may be derived from plasmids such as pBR322; pUC18; etc.

Such retroviral vectors are transfected or transduced into a packaging cell line, whereby there are generated infectious vector particles which include the retroviral vector. In general, the vector is transfected into the packaging cell line along with a packaging defective helper virus which includes genes encoding the gag and pol, and the env proteins of the virus. Representative examples of packaging cell lines include, but are not limited to, the PE501 and PA317 cell lines disclosed in Miller, et al., Biotechniques, Vol. 7 pgs. 980-990 (1989).

The vector particles generated from the packaging cell line. which are also engineered with a protein containing a receptor binding region that binds to a receptor of a target cell, said receptor being other than the amphotropic cell receptor, are targetable, whereby the receptor binding region enables the vector particles to bind to a target cell. The retroviral vector particles thus may be directly administered to a desired target cell ex vivo, and such cells may then be administered to a patient as part of a gene therapy procedure.

Although the vector particles may be administered directly to a target cell, the vector particles may be engineered such that the vector particles are "injectable" as well as targetable; i.e., the vector particles are resistant to inactivation by human serum, and thus the targetable vector particles may be administered to a patient by intravenous injection, and travel directly to a desired target cell or tissue without being inactivated by human serum.

The envelope of retroviruses also includes a protein known as p15E, and Applicants have found that retroviruses are susceptible to inactivation by human serum as a result of the action of complement proteins present in serum on the p15E protein portion of the retrovirus. Applicants have further found that such retroviruses can be made resistant to inactivation by human serum by mutating such p15E protein.

In one embodiment, therefore, the retroviral vector is engineered such that a portion of the DNA (RNA) encoding p15E protein (shown in the accompanying sequence listing as SEQ ID NO:7), has been mutated to render the vector particle resistant to inactivation by human serum; i.e., at least one amino acid but not all of the amino acids of the p15E protein has been changed. or mutated.

p15E protein is a viral protein having 196 amino acid residues. In viruses, sometimes all 196 amino acid residues are present, and in other viruses, amino acid residues 181 to 196 (known as the "r" peptide), are not present, and the resulting protein is the "mature" form of p15E known as p12E. Thus, viruses can contain both the p15E and p12E proteins. p15E protein is anchored in the viral membrane such that amino acid residues residues 1 to 134 are present on the outside of the virus. Although this embodiment of the present invention is not to be limited to any of the following reasoning, Applicants believe complement proteins may bind to this region whereby such binding leads to inactivation and/or lysis of the retrovirus. In particular, the p15E protein includes two regions, amino acid residues 39 to 61 (sometimes hereinafter referred to as region 1), and amino acid residues 101 to 123 (sometimes hereinafter referred to as region 2), which Applicants believe have an external location in the three-dimensional structure of the p15E protein; i.e., such regions are directly exposed to human serum. Region 2 is a highly conserved region in many retroviruses, even though the amino acid sequences of this region are not identical in all retroviruses. Such regions are complement binding regions. Examples of complement proteins which may bind to the complement binding regions are C1S and C1Q, which bind to regions 1 and 2.

In order to inactivate the retrovirus, complement proteins bind to both region 1 and region 2. Thus, in a preferred embodiment, at least one portion of DNA encoding a complement binding region of p15E protein has been mutated. Such a mutation results in a change of at least one amino acid residue of a complement binding region of p15E protein. The change in at least one amino acid residue of a complement binding region of p15E protein prevents binding of a complement protein to the complement binding region, thereby preventing complement inactivation of the retrovirus. In one embodiment, at least one amino acid residue in both complement binding regions of p15E protein is changed, whereas in another embodiment, at least one amino acid residue in one of the complement binding regions is changed.

It is to be understood, however, that the entire DNA sequence encoding p15E protein cannot be mutated because such a change renders the vectors unsuitable for in vivo use.

In one embodiment, the mutation of DNA (RNA) encoding p15E protein may be effected by deleting a portion of the p15E gene. and replacing the deleted portion of the p15E gene, with fragment(s) or portion(s) of a gene encoding another viral protein. In one embodiment, one portion of DNA encoding the p15E protein is replaced with a fragment of the gene encoding the p21 protein, which is an HTLV-I transmembrane protein. HTLV-I virus has been found to be resistant to binding by complement proteins and thus HTLV-I is resistant to inactivation by human serum (Hoshino, et al., Nature, Vol. 310, pgs. 324-325 (1984)). Thus. in one embodiment, there is also provided a retroviral vector particle wherein a portion of the p15E protein has been deleted and replaced with a portion of another viral protein, such as a portion of the p21 protein.

p21 protein (as shown in the accompanying sequence listing as SEQ ID NO:8) is a protein having 176 amino acid residues, and which, in relation to p15E, has significant amino acid sequence homology. In one embodiment, at least amino acid residues 39 to 61, and 101 to 123 are deleted from p15E protein, and replaced with amino acid residues 34 to 56 and 96 to 118 of p21 protein. In one alternative, at least amino acid residues 39 to 123 of p15E protein are deleted and replaced with amino acid residues 34 to 118 of p21 protein.

In another embodiment, amino acid residues 39 to 69 of p15E protein are deleted and replaced with amino acid residues 34 to 64 of p21 protein, and amino acid residues 96 to 123 of p15E protein are deleted and replaced with amino acid residues 91 to 118 of p21 protein.

Vector particles generated from such packaging lines, therefore, are "targetable" and "injectable," whereby such vector particles, upon administration to a patient, travel directly to a desired target cell or tissue.

The targetable vector particles are useful for the introduction of desired heterologous genes into target cells ex vivo. Such cells may then be administered to a patient as a gene therapy procedure, whereas vector particles which are targetable and injectable may be administered in vivo to the patient, whereby the vector particles travel directly to a desired target cell.

Thus, preferably, the vectors or vector particles of the present invention further include at least one heterologous gene. Heterologous or foreign genes which may be placed into the vector or vector particles include, but are not limited to, genes which encode cytokines or cellular growth factors, such as lymphokines, which are growth factors for lymphocytes. Other examples of foreign genes include, but are not limited to, genes encoding Factor VIII, Factor IX, tumor necrosis factors (TNF's), ADA, ApoE, ApoC, and Protein C.

The vectors of the present invention include one or more promoters. Suitable promoters which may be employed include, but are not limited to, the retroviral LTR; the SV40 promoter; and the human cytomegalovirus (CMV) promoter described in Miller, et al., Biotechniques, Vol. 7, No. 9, pgs 980-990 (1989), or any other promoter (e.g., cellular promoters such as eukaryotic cellular promoters including, but not limited to, the histone, pol III, and B-actin promoters). Other viral promoters which may be employed include, but are not limited to, adenovirus promoters, TK promoters, and B19 parvovirus promoters. The selection of a suitable promoter will be apparent to those skilled in the art from the teachings contained herein.

The vectors of the present invention may contain regulatory elements, where necessary to ensure tissue specific expression of the desired heterologous gene(s), and/or to regulate expression of the heterologous gene(s) in response to cellular or metabolic signals.

Although the invention has been described with respect to retroviral vector particles, other viral vector particles (such as, for example, adenovirus, adeno-associated virus, and Herpes Simplex virus particles), or synthetic particles may be constructed such that the vector particles include a receptor binding region that binds to a receptor of a target cell, wherein the receptor of a human target cell is other than the amphotropic cell receptor. Such vector particles are suitable for in vivo administration to a desired target cell.

Advantages of the present invention include the ability to provide vector particles which may be administered directly to a desired target cell or tissues, whereby desired genes are delivered to the target cell or tissue, whereby the target cell or tissue may produce the proteins expressed by such genes.

This invention will now be described with respect to the following examples; however, the scope of the present invention is not intended to be limited thereby.

### Example 1

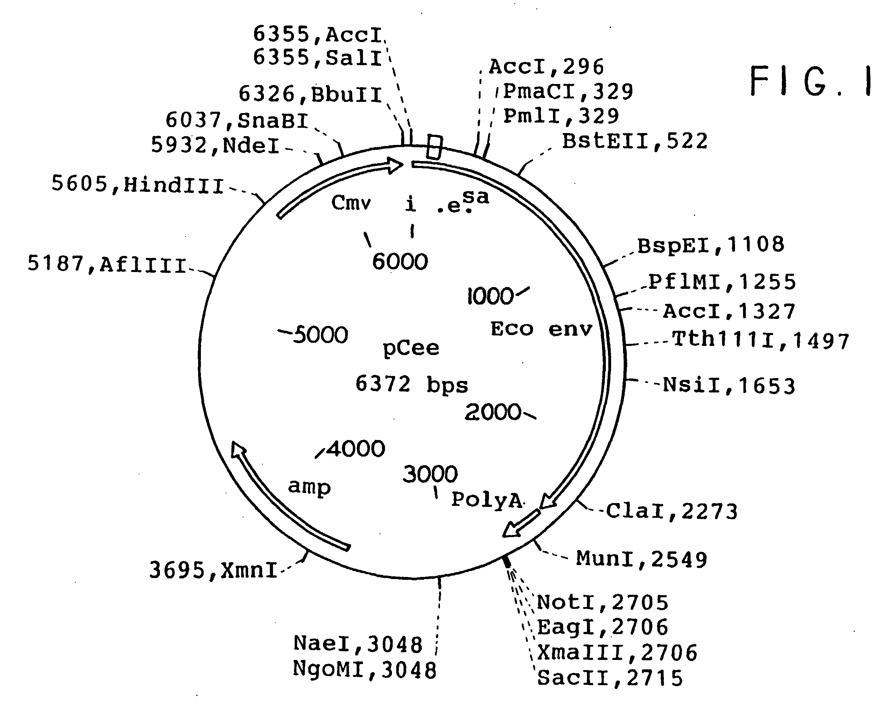

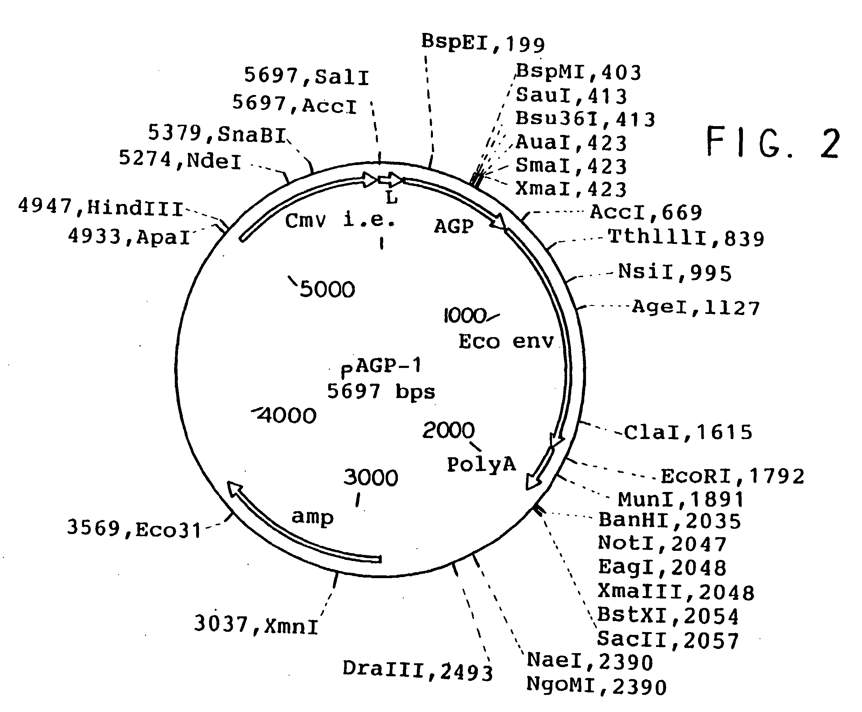

Plasmid pCee (Figure 1), which contains the ecotropic murine leukemia virus gp70 and p15E genes under the control of a CMV promoter, was cut with AccI, and an AccI fragment encoding amino acid residues 1-312 of the eco gp70 protein was removed. Cloned into the AccI site was a PCR fragment containing the eco gp70 secretion signal (or leader, which includes amino acid residues 1-33 of eco gp70), followed by mature rabbit alpha-1 acid glycoprotein (amino acid residues 19-201) (Ray, et al., Biochemical and Biophysical Research Communications, Vol. 178, No. 2, pgs. 507-513 (1991)). The amino acid sequence of rabbit alpha-1 acid glycoprotein is shown in (SEQ ID NO:5), and the DNA sequence encoding therefor is shown in (SEQ ID NO:6). The resulting plasmid pAGP-1 (Figure 2) contains the eco gp70 leader sequence (amino acid residues 1-33 of eco gp70), a sequence encoding the mature rabbit alpha-1 acid glycoprotein (amino acid residues 19-201), and a sequence encoding amino acid residues 313 to 469 of eco gp70.

### Example 2

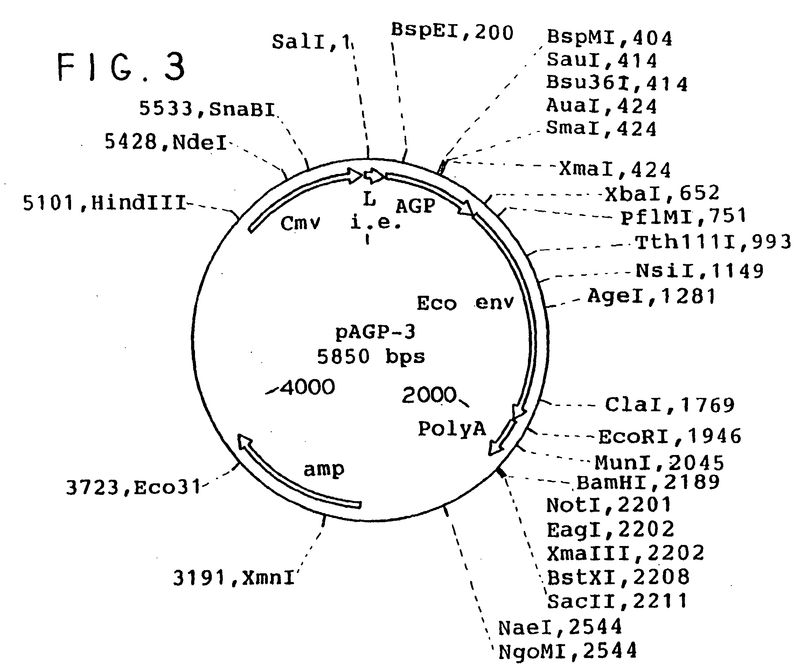

Plasmid pCee was cut with SalI and PflMI, and a SalI-PflMI fragment encoding amino acid residues 1-262 of eco gp70 was removed. Cloned into this site was a PCR generated SalI-PflMI fragment containing the eco gp70 leader sequence and the sequence encoding mature rabbit alpha-1 acid glycoprotein. The resulting plasmid, pAGP-3 (Figure 3) thus includes a sequence encoding the leader sequence of eco gp70, a sequence encoding mature rabbit alpha-1 acid glycoprotein; and a sequence encoding amino acid residues 263 to 469 of eco gp70.

### Example 3

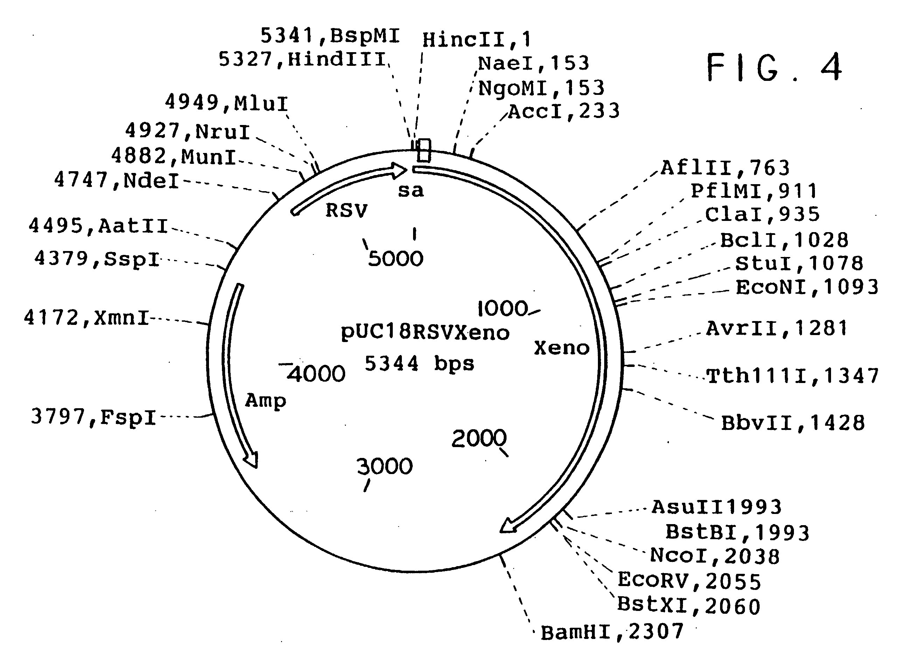

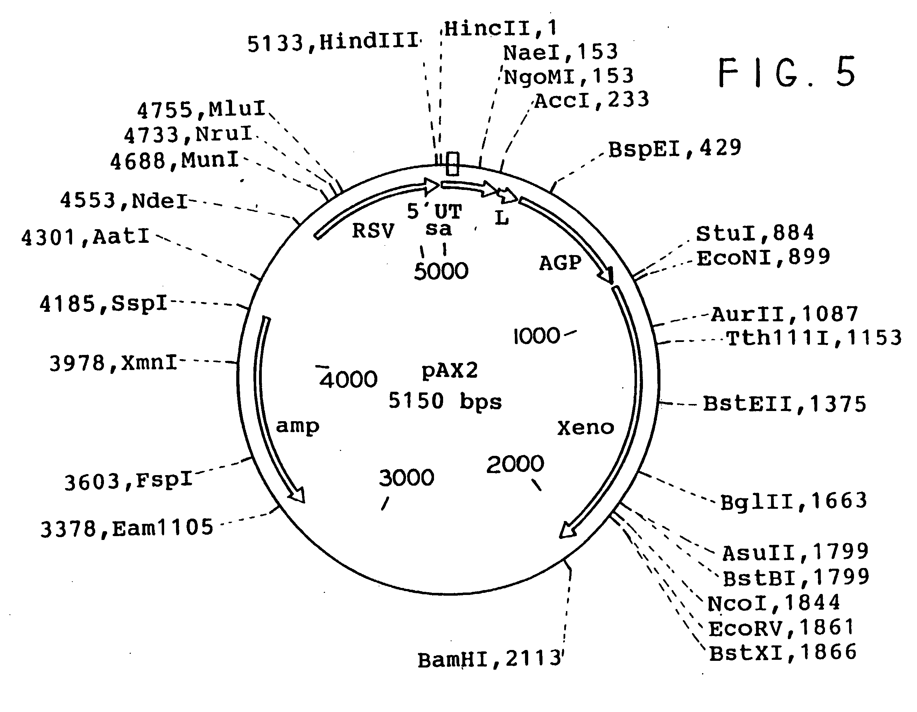

Plasmid pUC18RSVXeno (Figure 4), which contains the xenotrophic murine leukemia virus gp70 and p15E genes under the control of an RSV promoter, was cut with AccI and StuI, and an AccI-StuI fragment encoding amino acid residues 1-258 of xeno gp70 was removed. Cloned into this site was a PCR generated AccI-StuI fragment encoding the xeno gp70 leader (amino acid residues 1-30), and the mature rabbit alpha-1 acid glycoprotein. The resulting plasmid, pAX2 (Figure 5), thus contains a sequence encoding the xeno gp70 leader, a sequence encoding the mature rabbit alpha-1 acid glycoprotein, and amino acid residues 259-443 of xeno gp70.

### Example 4

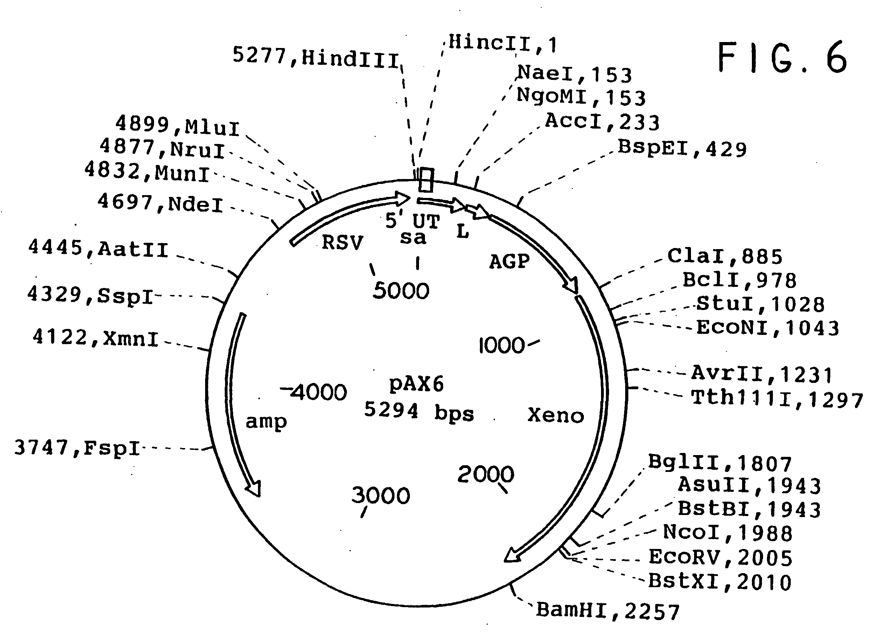

Plasmid pUC18RSVXeno was cut with AccI and ClaI, and a fragment encoding amino acid residues 1-210 of xeno gp70 was removed. Cloned into this site was a PCR generated AccI-ClaI fragment encoding the xeno gp70 leader, followed by mature rabbit alpha-1 acid glycoprotein. The resulting plasmid, pAX6 (Figure 6), thus includes a sequence encoding the xeno gp70 leader, a sequence encoding mature rabbit alpha-1 acid glycoprotein, and amino acid residues 211-443 of xeno gp70.

### Example 5

5x10⁵ GPL cells on 10 cm tissue culture plates were transfected (using CaPO₄) with 30 µg/plate of one of plasmids pAGP-1, pAGP-3, pAX2, or pAX6. The CaPO₄ is removed 24 hours later and 10 ml of fresh D10 medium is added for another 24 hours. The D10 medium is then removed and replaced with serum free DX medium for another 24 hours. The DX medium is then collected, filtered, and stored on ice. This supernatant contains the vector particles.

The supernatants were then filtered and collected by standard procedures and then centrifuged. After centrifugation. the virus pellets were reconstituted in a buffer containing 0.1M sodium acetate, 0.15M sodium chloride, and 2mM calcium chloride; the buffer was sterilized using a Falcon 0.2 millimicron tissue culture filter.

2.2 ml of concentrated supernatant containing viral particles generated from pAGP-1 or pAGP-3, said viral particles sometimes hereinafter referred to as Chimeric-1 or Chimeric-3, were loaded onto two disposable plastic columns which were alcohol sterilized and dried. To each column (1cm x 6cm), one unit of neuraminidase from Clostridium perfringens which was bound to beaded agarose was added as a 2 ml suspension. This represents 1 ml of packed gel or unit of enzyme per column (15.7 mg of agarose/ml and 28 units per gram of agarose). A unit is defined as the amount of neuraminidase which will liberate 1.0 micromole of N-acetylneuraminic acid per minute from NAN-lactose at pH 5.0 and 37°C.

The columns were then washed with a large excess (50 ml) of the buffer hereinabove described to free the resin of all traces of free neuraminidase and to sterilize the resin prior to incubation with virus. The columns were then dried, and the bottoms were sealed with caps and secured with parafilm. The concentrated virus which was reconstituted in the buffer (2.0 ml per sample) was then added to the resin. The tops were placed on the columns and secured with parafilm. The resin was gently re-suspended by hand. The virus was then incubated with the resin for 1 hour at room temperature with gentle rotation on a wheel. The columns were checked periodically to ensure good mixing of resin and virus.

At the end of the incubation period, the Chimera-1 and Chimera-3 viruses were recovered by gentle vacuum filtration and collected into separate sterile 12x75 mm plastic polypropylene Falcon 2063 tubes. Recovery was greater than 90%, giving about 1.8 ml of desialated virus.

6-well plates containing about 10⁵ receptor-positive (Hep G2) or receptor-negative (SK HepI) human hepatocytes in 2 ml D10 media were employed as target cells. 24 hours after the cells were plated, 1 ml of D10 was removed from the first well and 2 mi of neuraminidase-treated (or untreated as a control) viral supernatant containing Chimeric-1 or Chimeric-3 was added and mixed well. 200 ul from the first well was diluted into the 2 ml present in the second well, was then mixed; and then 200 ul from the second well was diluted into the 1.8 ml present in the third well, thereby giving approximate dilutions of 2/3, 1/15, and 1/150. 8 ug/ml of Polybrene was included in each well during the transduction. The viral particles were left in contact with the cells overnight, followed by removal of media containing viral particles, and replaced with D10 containing 1,000 mg/ml of G418. The medium was changed with fresh D10 and G418 every 4 to 5 days as necessary. G418-resistant colonies were scored after 2 to 3 weeks.

### Example 6

The pre-packaging cell line GP8, which expresses the retroviral proteins gag and pol, and the packaging cell lines derived from them which also express the chimeric gp70 glycoproteins encoded by the plasmids pAGP-1, pAGP-3, pAX2, or pAX6 were maintained in cell culture and exposed to successively higher concentrations of wheat germ agglutinin; starting with 15 ug/ml. The cell lines were maintained under WGA selection in cell culture for 6 to 8 weeks until populations resistant to 40-50 ug/ml WGA were obtained. The latter were then subjected to fluoresence-activated cell sorting using FITC-conjugated lectins to enrich for the cells expressing the desired mutant glycotype (e.g., FITC-Erythrina Cristagalli agglutinin for beta-D-galactosyl groups, and FITC-concanavalin A for alpha-D-mannosyl groups). Retroviral vector packaging and producer cell lines were then generated from the resulting populations by standard techniques.

It is to be understood, however, that the scope of the present invention is not to be limited to the specific embodiments described above. The invention may be practiced other than as particularly described and still be within the scope of the accompanying claims.

### SEQUENCE LISTING

(1) GENERAL INFORMATION:
   (i) APPLICANT: Anderson, W. French
      Baltrucki, Leon F.
      Mason, James M.
   (ii) TITLE OF INVENTION: Targetable Vector Particles
   (iii) NUMBER OF SEQUENCES: 8
   (iv) CORRESPONDENCE ADDRESS:
      (A) ADDRESSEE: Carella, Byrne, Bain, Gilfillan, Cecchi & Stewart
      (B) STREET: 6 Becker Farm Road
      (C) CITY: Roseland
      (D) STATE: New Jersey
      (E) COUNTRY: USA
      (F) ZIP: 07068
   (v) COMPUTER READABLE FORM:
      (A) MEDIUM TYPE: 3.5 inch diskette
      (B) COMPUTER: IBM PS/2
      (C) OPERATING SYSTEM: PC-DOS
      (D) SOFTWARE: DW4.V2
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: Lillie, Raymond J.
      (B) REGISTRATION NUMBER: 31,778
      (C) REFERENCE/DOCKET NUMBER: 271010-107
   (ix) TELECOMMUNICATION INFORMATION:
      (A) TELEPHONE: 201-994-1700
      (B) TELEFAX: 201-994-1744
(2) INFORMATION FOR SEQ ID NO: 1:
   (i) SEQUENCE CHARACTERISTICS:
      (A) LENGTH: 469 bases
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Ecotropic gp70 Protein
   (xi) SEQUENCE DESCRIPTION: SEQ ID NO:1:
   Met Ala Arg Ser Thr Leu Ser Lys Pro Leu
                       5                   10
   Lys Asn Lys Val Asn  Pro Arg Gly Pro Leu
                      15                   20
   Ile Pro Leu Ile Leu  Leu Met Leu Arg Gly
                      25                   30
   Val Ser Thr Ala Ser  Pro Gly Ser Ser Pro
                      35                   40
   His Gly Val Tyr Asn  Ile Thr Trp Glu Val
                      45                   50
   Thr Asn Gly Asp Arg  Glu Thr Val Trp Ala
                      55                   60
   Thr Ser Gly Asn His  Pro Leu Trp Thr Trp
                      65                   70
   Trp Pro Asp Leu Thr  Pro Asp Leu Cys Met
                      75                   80
   Leu Ala His His Gly  Pro Ser Tyr Trp Gly
                      85                   90
   Leu Glu Tyr Gln Ser  Pro Phe Ser Ser Pro
                      95                  100
   Pro Gly Pro Pro Cys  Cys Ser Gly Gly Ser
                       105                     110
   Ser Pro Gly Cys Ser  Arg Asp Cys Glu Glu
                       115                     120
   Pro Leu Thr Ser Leu  Thr Pro Arg Cys Asn
                       125                     130
   Thr Ala Trp Asn Arg  Leu Lys Leu Asp Gln
                       135                     140
   Thr Thr His Lys Ser  Asn Glu Gly Phe Tyr
                       145                     150
   Val Cys Pro Gly Pro  His Arg Pro Arg Glu
                       155                     160
   Ser Lys Ser Cys Gly  Gly Pro Asp Ser Phe
                       165                     170
   Tyr Cys Ala Tyr Trp  Gly Cys Glu Thr Thr
                       175                     180
   Gly Arg Ala Tyr Trp  Lys Pro Ser Ser Ser
                       185                     190
   Trp Asp Phe Ile Thr  Val Asn Asn Asn Leu
                       195                     200
   Thr Ser Asp Gln Ala  Val Gln Val Cys Lys
                       205                     210
   Asp Asn Lys Trp Cys  Asn Pro Leu Val Ile
                       215                     220
   Arg Phe Thr Asp Ala  Gly Arg Arg Val Thr
                       225                     230
   Ser Trp Thr Thr Gly  His Tyr Trp Gly Leu
                       235                     240
   Arg Leu Tyr Val Ser  Gly Gln Asp Pro Gly
                       245                     250
   Leu Thr Phe Gly Ile  Arg Leu Arg Tyr Gln
                       255                     260
   Asn Leu Gly Pro Arg  Val Pro Ile Gly Pro
                       265                     270
   Asn Pro Val Leu Ala  Asp Gln Gln Pro Leu
   275 280
   Ser Lys Pro Lys Pro Val Lys Ser Pro Ser
   285 290
   Val Thr Lys Pro Pro Ser Gly Thr Pro Leu
   295 300
   Ser Pro Thr Gln Leu Pro Pro Ala Gly Thr
   305 310
   Glu Asn Arg Leu Leu Asn Leu Val Asp Gly
   315 320
   Ala Tyr Gln Ala Leu Asn Leu Thr Ser Pro
   325 330
   Asp Lys Thr Gln Glu Cys Trp Leu Cys Leu
   335 340
   Val Ala Gly Pro Pro Tyr Tyr Glu Gly Val
   345 350
   Ala Val Leu Gly Thr Tyr Ser Asn His Thr
   355 360
   Ser Ala Pro Ala Asn Cys Ser Val Ala Ser
   365 370
   Gln His Lys Leu Thr Leu Ser Glu Val Thr
   375 380
   Gly Gln Gly Leu Cys Ile Gly Ala Val Pro
   385 390
   Lys Thr His Gln Ala Leu Cys Asn Thr Thr
   395 400
   Gln Thr Ser Ser Arg Gly Ser Tyr Tyr Leu
   405 410
   Val Ala Pro Thr Gly Thr Met Trp Ala Cys
   415 420
   Ser Thr Gly Leu Thr Pro Cys Ile Ser Thr
   425 430
   Thr Ile Leu Asn Leu Thr Thr Asp Tyr Cys
   435 440
   Val Leu Val Glu Leu Trp Pro Arg Val Thr
                       445                 450
   Tyr His Ser Pro Ser  Tyr Val Tyr Gly Leu
                       455                 460
   Phe Glu Arg Ser Asn  Arg His Lys Arg
                       465
(2) INFORMATION FOR SEQ ID NO: 2:
   (i) SEQUENCE CHARACTERISTICS
      (A) LENGTH: 1446 bases
      (B) TYPE: nucleic acid
      (C) STRANDEDNESS: single
      (D) TOPOLOGY: linear
   (ii) MOLECULE TYPE: viral DNA
   (xi) SEQUENCE DESCRIPTION: SEQ ID NO:2:
   GGCTGCCGAC CCCGGGGGTG GACCATCCTC TAGACTGACA TGGCGCGTTA AACGCTCTCA     60
   AAACCCCTTA AAAATAAGGT TAACCCGCGA GGCCCCCTAA TCCCCTTAAT TCTTCTGATG    120
   CTCAGAGGGG TCAGTACTGC TTCGCCCGGC TCCAGTCCTC ATCAAGTCTA TAATATCACC    180
   TGGGAGGTAA CCAATGGAGA TCGGGAGACG GTATGGGCAA CTTCTGGCAA CCACCCTCTG    240
   TGGACCTGGT GGCCTGACCT TACCCCAGAT TTATGTATGT TAGCCCACCA TGGACCATCT    300
   TATTGGGGGC TAGAATATCA ATCCCCTTTT TCTTCTCCCC CGGGGCCCCC TTGTTGCTCA    360
   GGGGGCAGCA GCCCAGGCTG TTCCAGAGAC TGCGAAGAAC CTTTAACCTC CCTCACCCCT    420
   CGGTGCAACA CTGCCTGGAA CAGACTCAAG CTAGACCAGA CAACTCATAA ATCAAATGAG    480
   GGATTTTATG TTTGCCCCGG GCCCCACCGC CCCCGAGAAT CCAAGTCATG TGGGGGTCCA    540
   GACTCCTTCT ACTGTGCCTA TTGGGGCTGT GAGACAACCG GTAGAGCTTA CTGGAAGCCC    600
   TCCTCATCAT GGGATTTCAT CACAGTAAAC AACAATCTCA CCTCTGACCA GGCTGTCCAG    660
   GTATGCAAAG ATAATAAGTG GTGCAACCCC TTAGTTATTC GGTTTACAGA CGCCGGGAGA    720
   CGGGTTACTT CCTGGACCAC AGGACATTAC TGGGGCTTAC GTTTGTATGT CTCCGGACAA    780
   GATCCAGGGC TTACATTTGG GATCCGACTC AGATACCAAA ATCTAGGACC CCGCGTCCCA    840
   ATAGGGCCAA ACCCCGTTCT GGCAGACCAA CAGCCACTCT CCAAGCCCAA ACCTGTTAAG    900
   TCGCCTTCAG TCACCAAACC ACCCAGTGGG ACTCCTCTCT CCCCTACCCA ACTTCCACCG    960
   GCGGGAACGG AAAATAGGCT GCTAAACTTA GTAGACGGAG CCTACCAAGC CCTCAACCTC   1020
   ACCAGTCCTG ACAAAACCCA AGAGTGCTGG TTGTGTCTAG TAGCGGGACC CCCCTACTAC   1080
   GAAGGGGTTG CCGTCCTGGG TACCTACTCC AACCATACCT CTGCTCCAGC CAACTGCTCC   1140
   GTGGCCTCCC AACACAAGTT GACCCTGTCC GAAGTGACCG GACAGGGACT CTGCATAGGA   1200
   GCAGTTCCCA AAACACATCA GGCCCTATGT AATACCACCC AGACAAGCAG TCGAGGGTCC   1260
   TATTATCTAG TTGCCCCTAC AGGTACCATG TGGGCTTGTA GTACCGGGCT TACTCCATGC   1320
   ATCTCCACCA CCATACTGAA CCTTACCACT GATTATTGTG TTCTTGTCGA ACTCTGGCCA   1380
   AGAGTCACCT ATCATTCCCC CAGCTATGTT TACGGCCTGT TTGAGAGATC CAACCGACAC   1440
   AAAAGA                                                              1446
(2) INFORMATION FOR SEQ ID NO: 3:
   (i) SEQUENCE CHARACTERISTICS:
      (A) LENGTH: 443 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY:xenotropie gp70 protein
   (xi) SEQUENCE DESCRIPTION: SEQ ID NO:3:
(2) INFORMATION FOR SEQ ID NO: 4:
   (i) SEQUENCE CHARACTERISTICS
      (A) LENGTH: 1356 bases
      (B) TYPE: nucleic acid
      (C) STRANDEDNESS: single
      (D) TOPOLOGY: linear
   (ii) MOLECULE TYPE: viral DNA
   (xi) SEQUENCE DESCRIPTION: SEQ ID NO:4:
   GCGACAACTC CTCCAGCCGG GAACAGCATG GAAGGTTCAG CGTTCTCAAA ACCCCTTAAA     60
   GATAAGATTA ACCCGTGGGG CCCCCTAATA GTTATGGGGA TCTTGGTGAG GGCAGGAGCT    120
   TCGGTACAAC GTGACAGCCC TCACCAGATC TTCAATGTTA CTTGGAGAGT TACCAACCTA    180
   ATGACAGGAC AAACAGCTAA CGCCACCTCC CTCCTGGGGA CGATGACAGA CACCTTCCCT    240
   AAACTATATT TTGACCTGTG TGATTTAGTA GGAGACTACT GGGATGACCC AGAACCCGAT    300
   ATTGGGGATG GTTGCCGCAC TCCCGGGGGA AGAAGAAGGA CAAGACTGTA TGACTTCTAT    360
   GTTTGCCCCG GTCATACTGT ACCAATAGGG TGTGGAGGGC CGGGAGAGGG CTACTGTGGC    420
   AAATGGGGAT GTGAGACCAC TGGACAGGCA TACTGGAAGC CATCATCATC ATGGGACCTA    480
   ATTTCCCTTA AGCGAGGAAA CACTCCTAAG GATCAGGGCC CCTGTTATGA TTCCTCGGTC    540
   TCCAGTGGCG TCCAGGGTGC CACACCGGGG GGTCGATGCA ACCCCCTGGT CTTAGAATTC    600
   ACTGACGCGG GTAGAAAGGC CAGCTGGGAT GCCCCCAAAG TTTGGGGACT AAGACTCTAT    660
   CGATCCACAG GGGCCGACCC GGTGACCCGG TTCTCTTTGA CCCGCCAGGT CCTCAATGTA    720
   GGACCCCGCG TCCCCATTGG GCCTAATCCC GTGATCACTG ACCAGCTACC CCCATCCCAA    780
   CCCGTGCAGA TCATGCTCCC CAGGCCTCCT CATCCTCCTC CTTCAGGCAC GGTCTCTATG    840
   GTACCTGGGG CTCCCCCGCC TTCTCAACAA CCTGGGACGG GAGACAGGCT GCTAAATCTG    900
   GTAGAAGGAG CCTACCAAGC ACTCAACCTC ACCAGTCCTG ACAAAACCCA AGAGTGCTGG    960
   TTGTGTCTGG TATCGGGACC CCCCTACTAC GAAGGGCTTG CCGTCCTAGG TACCTACTCC   1020
   AACCATACCT CTGCCCCAGC TAACTGCTCC GTGGCCTCCC AACACAAGCT GACCCTGTCC   1080
   GAAGTAACCG GACAGGGACT CTGCGTAGGA GCAGTTCCCA AAACCCATCA GGCCCTGTGT   1140
   AATACCACCC AGAAGACGAG CGACGGGTCC TACTATCTGG CTGCTCCCGC CGGGACCATC   1200
   TGGGCTTGCA ACACCGGGCT CACTCCCTGC CTATCTACTA CTGTACTCAA CCTCACCACC   1260
   GATTACTGTG TCCTGGTTGA GCTCTGGCCA AAGGTAACCT ACCACTCCCC TGATTATGTT   1320
   TATGGCCAGT TTGAAAAGAA AACTAAATAT AAAAGA                             1356
(2) INFORMATION FOR SEQ ID NO: 5:
   (i) SEQUENCE CHARACTERISTICS
      (A) LENGTH: 201 amino acids
      (B) TYPE: amino acid
      (C) STRANDEDNESS:
      (D) TOPOLOGY: linear
   (ii) MOLECULE TYPE: protein
   (ix) FEATURE
      (A) NAME/KEY:rabbit alpha-1-acid glycoprotein
   (x) PUBLICATION INFORMATION
      (A) AUTHOR Ray, et al.
      (B) TITLE:
      (C) JOURNAL: Biochem. and Biophys. Res. Comm.
      (D) VOLUME: 178
      (E) ISSUE: No. 2
      (F) PAGES: 507-513
      (G) DATE: 1991
   (xi) SEQUENCE DESCRIPTION: SEQ ID NO: 5:
   Met Ala Leu Pro Trp  Ala Leu Ala Val Leu
                     5                   10
   Ser Leu Leu Pro Leu  Leu His Ala Gln Asp
                    15                   20
   Pro Ala Cys Ala Asn  Phe Ser Thr Ser Pro
                    25                   30
   Ile Thr Asn Ala Thr Leu Asp Gln Leu Ser
   35 40
   His Lys Trp Phe Phe Thr Ala Ser Ala Phe
   45 50
   Arg Asn Pro Lys Tyr Lys Gln Leu Val Gln
   55 60
   His Thr Gln Ala Ala Phe Phe Tyr Phe Thr
   65 70
   Ala Ile Lys Glu Glu Asp Thr Leu Leu Leu
   75 80
   Arg Glu Tyr Ile Thr Thr Asn Asn Thr Cys
   85 90
   Phe Tyr Asn Ser Ser Ile Val Arg Val Gln
   95 100
   Arg Glu Asn Gly Thr Leu Ser Lys His Asp
   105 110
   Gly Ile Arg Asn Ser Val Ala Asp Leu Leu
   115 120
   Leu Leu Arg Asp Pro Gly Ser Phe Leu Leu
   125 130
   Val Phe Phe Ala Gly Lys Glu Gln Asp Lys
   135 140
   Gly Met Ser Leu Tyr Thr Asp Lys Pro Lys
   145 150
   Ala Ser Thr Glu Gln Leu Glu Glu Phe Tyr
   155 160
   Glu Ala Leu Thr Cys Leu Gly Met Asn Lys
                   165                 170
   Thr Glu Val Val Tyr Thr Asp Trp Thr Lys
                   175                 180
   Asp Leu Cys Glu Pro Leu Glu Lys Gln His
                   185                 190
   Glu Glu Glu Arg Lys Lys Glu Lys Ala Glu
                   195                 200
   Ser
(2) INFORMATION FOR SEQ ID NO: 6
   (i) SEQUENCE CHARACTERISTICS
      (A) LENGTH: 759 bases
      (B) TYPE: nucleic acid
      (C) STRANDEDNESS: single
      (D) TOPOLOGY: linear
   (ii) MOLECULE TYPE: genomic DNA
   (x) PUBLICATION INFORMATION:
      (A) AUTHOR Ray, et al.
      (B) TITLE:
      (C) JOURNAL: Biochem. and Biophys. Res. Comm.
      (D) VOLUME: 178
      (E) ISSUE: NO. 2
      (F) PAGES: 507-513
      (G) DATE: 1991
   (xi) SEQUENCE DESCRIPTION: SEQ ID NO:6:
   AGCTCTGCCT GGCTCCAGCG CCTCTGTGTC TCAGCATGGC CCTGCCCTGG GCCCTCGCCG 60
   TCCTGAGCCT CCTCCCTCTG CTGCATGCCC AGGACCCAGC GTGTGCCAAC TTCTCGACCA 120
   GCCCTATCAC CAATGCCACC CTGGACCAGC TCTCCCACAA GTGGTTTTTT ACCGCCTCGG 180
   CCTTCCGGAA CCCCAAGTAC AAGCAGCTGG TGCAGCATAC CCAGGCGGCC TTTTTCTACT 240
   TCACCGCCAT CAAAGAGGAG GACACCTTGC TGCTCCGGGA GTACATAACC ACGAACAACA 300
   CGTGCTTCTA TAACTGCAGC ATCGTGAGGG TCCAGAGAGA GAATGGGACC CTCTCCAAAC 360
   ACGACGGCAT ACGAAATAGC GTGGCCGACC TGCTGCTCCT CAGGGACCCC GGGAGCTTCC 420
   TCCTCGTCTT CTTCGCTGGG AAGGAGCAGG ACAAGGGAAT GTCCTTCTAC ACCGACAAGC 480
   CCAAGGCCAG CCCGGAACAA CTGGAAGAGT TCTACGAAGC CCTCACGTGC CTGGGCATGA 540
   ACAAGACGGA AGTCGTCTAC ACTGACTGGA CAAAGGATCT GTGCGAGCCG CTGGAGAAGC 600
   AACACGAGGA GGAGAGGAAG AAGGAAAAGG CAGAGTCATA GGGCACAGCA CCGGCTCCGG 660
   GACTCGGGGC CCACCCCCTG CACCTGCCTT TTTGTTTGTT TTGTAAATCT CTGTTCTTTC 720
   CCATGGTTGC ATCAATAAAA CTGCTGGACC AGTAAAAAA 759
(2) INFORMATION FOR SEQ ID NO: 7:
   (i) SEQUENCE CHARACTERISTICS:
      (A) LENGTH: 196 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: ecotropic p15E protein.
   (xi) SEQUENCE DESCRIPTION; SEQ ID NO: 7: INFORMATION FOR SEQ ID NO:8:
   (i) SEQUENCE CHARACTERISTICS:
      (A) LENGTH: 176 amino acids
      (B) TYPE: amino acid
      (C) STRANDEDNESS:
      (D) TOPOLOGY: linear
   (ii) MOLECULE TYPE: protein
   (ix) FEATURE:
      (A) NAME/KEY: HTLV-I p21 protein
   (x) PUBLICATION INFORMATION:
      (A) AUTHOR: Malik, et al.
      (B) TITLE:
      (C) JOURNAL: J. Gen. Virol.
      (D) VOLUME: 69
      (E) ISSUE:
      (F) PAGES: 1695-1710
      (G) DATE: 1988
   (xi) SEQUENCE DESCRIPTION: SEQ ID NO:8:

## Claims

1. A retroviral vector particle, said retroviral vector particle including a retroviral envelope protein, which includes a receptor binding domain, a hinge region and a body region, wherein a portion of said retroviral envelope protein is deleted and a receptor binding region or a ligand that binds to a receptor of a target cell is inserted into said deleted portion, said receptor of a target cell being other than the amphotropic cell receptor, and wherein the only portion of the retroviral envelope protein that is deleted is
(i) a portion of the receptor binding domain and a portion or all of the hinge region, or
(ii) all of the receptor binding domain and a portion or all of the hinge region.

2. The retroviral vector particle of claim 1 wherein a portion of the hinge region of the retroviral envelope protein is deleted.

3. The retroviral vector particle of claim 1 wherein said receptor binding region that binds to a receptor of a target cell is a receptor binding region of a human virus.

4. The retroviral vector particle of claim 3 wherein said receptor binding region of a human virus is a hepatitis B virus surface protein binding region and said target cell is a liver cell.

5. The retroviral vector particle of claim 3 wherein said receptor binding region of a human virus is the receptor binding region of gp46 of HTLV-I virus and said target cell is a T-cell.

6. The retroviral vector particle of claim 3 wherein said receptor binding region of a human virus is the HIV gp 120 CD4 binding region and said target cell is a T4 helper cell.

7. The retroviral vector particle of claim 1 wherein said receptor binding region or ligand that binds to a receptor of a target cell, which is inserted into said deleted portion is a protein which binds to an asialoglycoprotein receptor of hepatocytes.

8. The retroviral vector particle of claim 7 wherein said protein which binds to an asialoglycoprotein receptor of hepatocytes is alpha-1 acid glycoprotein.

9. The retroviral vector particle of claim 1 further including at least one heterologous gene.

10. A packaging cell line which produces the retroviral vector particles of any of precedent claims.

11. The use of a retroviral vector of any claims 1 to 9, for the manufacture of a therapeutic or prophylactic composition.

## Patentansprüche

1. Retrovirales Vektorpartikel, wobei das retrovirale Vektorpartikel ein retrovirales Hüllprotein umfasst, das eine Rezeptorbindedomäne, eine Gelenkregion und eine Kemregion enthält, wobei ein Teil des retroviralen Hüllproteins deletiert ist und eine Rezeptorbindungsregion oder ein Ligand, der an einen Rezeptor einer Zielzelle bindet, in diesen deletierten Teil inseriert ist, wobei der Rezeptor der Zielzelle ein anderer als der amphotrophe Zellrezeptor ist und worin der einzige Teil des retroviralen Hüllproteins, der deletiert ist, folgender ist
(i) ein Teil der Rezeptorbindungsdomäne und ein Teil oder die gesamte Gelenkregion, oder
(ii) die gesamte Rezeptorbindungsdomäne und ein Teil oder die gesamte Gelenkregion.

2. Retrovirales Vektorpartikel nach Anspruch 1, worin ein Teil der Gelenkregion des retroviralen Hüllproteins deletiert ist.

3. Retrovirales Vektorpartikel nach Anspruch 1, worin die Rezeptorbindungsregion, die an einen Rezeptor einer Zielzelle bindet, eine Rezeptorbindungsregion eines humanen Virus ist.

4. Retrovirales Vektorpartikel nach Anspruch 3, worin die Rezeptorbindungsregion eines humanen Virus eine Oberflächenproteinbinderegion des Hepatitis B Virus ist und die Zielzelle eine Leberzelle ist.

5. Retrovirales Vektorpartikel nach Anspruch 3, worin die Rezeptorbindungsregion eines humanen Virus die Rezeptorbindungsregion von gp46 des HTLV-I Virus ist und die Zielzelle eine T-Zelle ist.

6. Retrovirales Vektorpartikel nach Anspruch 3, worin die Rezeptorbindungsregion eines humanen Virus die CD4 Bindungsregion des HIV gp 120 ist und die Zielzelle eine T4 Helferzelle ist.

7. Retrovirales Vektorpartikel nach Anspruch 1, worin die Rezeptorbindungsregion oder der Ligand, der an einen Rezeptor einer Zielzelle bindet, welcher im deletierten Teil inseriert ist, ein Protein ist, das an einen Asialoglycoproteinrezeptor von Hepatocyten bindet.

8. Retrovirales Vektorpartikel nach Anspruch 7, worin das Protein, das an einen Asialoglycoproteinrezeptor von Hepatocyten bindet, das alpha-1 Säureglycoprotein ist.

9. Retrovirales Vektorpartikel nach Anspruch 1, das ferner zumindest ein heterologes Gen umfasst.

10. Verpackungszelllinie, die die retroviralen Partikel aller vorangehenden Ansprüche umfasst.

11. Verwendung eines retroviralen Vektors nach einem der Ansprüche 1 bis 9 zur Herstellung einer therapeutischen oder prophylaktischen Zusammensetzung.

## Revendications

1. Particule de vecteur rétroviral, ladite particule de vecteur rétroviral comprenant une protéine d'enveloppe rétrovirale, qui comprend un domaine de liaison au récepteur, une région charnière et une région de corps, dans laquelle une partie de ladite protéine d'enveloppe rétrovirale est délétée, et une région de liaison au récepteur ou un ligand qui se lie à un récepteur d'une cellule cible est inséré dans ladite partie délétée, ledit récepteur d'une cellule cible étant différent du récepteur cellulaire amphotrope, et dans laquelle la seule portion de la protéine d'enveloppe rétrovirale qui est délétée est :
(i) une partie du domaine de liaison au récepteur et une partie ou la totalité de la région charnière, ou
(ii) tout le domaine de liaison au récepteur et une partie ou la totalité de la région charnière.

2. Particule de vecteur rétroviral suivant la revendication 1, dans laquelle une partie de la région charnière de la protéine d'enveloppe rétrovirale est délétée.

3. Particule de vecteur rétroviral suivant la revendication 1, dans laquelle ladite région de liaison au récepteur qui se lie à un récepteur d'une cellule cible est une région de liaison au récepteur d'un virus humain.

4. Particule de vecteur rétroviral suivant la revendication 3, dans laquelle ladite région de liaison au récepteur d'un virus humain est une région de liaison à une protéine de surface du virus de l'hépatite B, et ladite cellule cible est une cellule hépatique.

5. Particule de vecteur rétroviral suivant la revendication 3, dans laquelle ladite région de liaison au récepteur d'un virus humain est la région de liaison au récepteur de gp46 du virus HTLV-1, et ladite cellule cible est une cellule T.

6. Particule de vecteur rétroviral suivant la revendication 3, dans laquelle ladite région de liaison au récepteur d'un virus humain est la région de liaison de gp 120 CD4 de HIV, et ladite cellule cible est une cellule auxiliaire T4.

7. Particule de vecteur rétroviral suivant la revendication 1, dans laquelle ladite région de liaison au récepteur ou ledit ligand qui se lie à un récepteur d'une cellule cible, qui est inséré dans ladite partie délétée, est une protéine qui se lie à un récepteur d'asialoglycoprotéine d'hépatocytes.

8. Particule de vecteur rétroviral suivant la revendication 7, dans laquelle ladite protéine qui se lie à un récepteur d'asialoglycoprotéine d'hépatocytes est une glycoprotéine acide alpha-1.

9. Particule de vecteur rétroviral suivant la revendication 1, comprenant en outre au moins un gène hétérologue.

10. Lignée cellulaire d'encapsulation qui produit les particules de vecteur rétroviral suivant l'une quelconque des revendications précédentes.

11. Utilisation d'un vecteur rétroviral suivant l'une quelconque des revendications 1 à 9, pour la fabrication d'une composition thérapeutique ou prophylactique.
